# EUROPÄISCHE PATENTANMELDUNG

(11) **EP 0 593 976 A1**
(43) Veröffentlichungstag der Anmeldung: **27.04.1994**
(21) Anmeldenummer: 93116152.5
(22) Anmeldetag: 06.10.1993
(51) Int. Cl.: C07D 215/14, A61K 31/47

(54) **Kristallines (R)-(-)-2-Cycloheptyl-N-methylsulfonyl-[4-(2-chinolinyl-methoxy)-phenyl]-acetamid**

(30) Priorität: 19.10.1992 DE 4235133
(71) Anmelder: BAYER AG, 51368 Leverkusen (DE)
(72) Erfinder: Grunenberg, Alfons, Dr., D-41539 Dormagen (DE); Mertens, Hubert, Dr., D-42799 Leichlingen (DE)

(57) **Zusammenfassung**

Der Leukotrien-Synthesehemmer (R)-(-)-2-Cycloheptyl-N-methylsulfonyl-[4-(2-chinolinyl-methoxy)-phenyl]-acetamid in kristalliner Form wird hergestellt, in dem der Wirkstoff in nichtkristalliner Form in inerten organischen Lösemitteln, gegebenenfalls in Anwesenheit von Wasser suspendiert wird und bei erhöhter Temperatur durch Rühren in die kristalline Modifikation überführt wird. Der kristalline Wirkstoff eignet sich insbesondere zur Herstellung von Arzneimitteln mit erhöhter Lagerstabilität und Temperaturbeständigkeit.

## Beschreibung

Die Erfindung betrifft eine kristalline Form des (R)-(-)-2-Cycloheptyl-N-methylsulfonyl-[4-(2-chinolinyl-methoxy)-phenyl]-acetamid, ein Verfahren zur Herstellung und dessen Verwendung in Arzneimitteln.

Der Leukotrien-Synthesehemmer (R)-(-)-2-Cycloheptyl-N-methylsulfonyl-[4-(2-chinolinyl-methoxy)-phenyl]-acetamid der Formel (I) 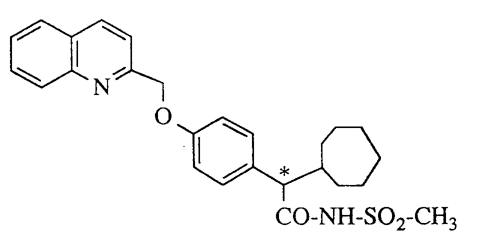
dessen Herstellung sowie dessen Verwendung in Arzneimitteln ist bereits in EP 344 519 beschrieben.

Nach dem dort beschriebenen Herstellverfahren erhält man die Verbindung der Formel (I) in Form eines nichtkristallinen Pulvers. Eine solvatfreie, kristalline Modifikation ist bisher nicht bekannt.

Es hat sich jedoch gezeigt, daß die nichtkristalline Form der Verbindung (I), insbesondere bei der Herstellung von festen Arzneimitteln entscheidende Nachteile hat. So weisen beispielsweise Arzneimittel, die die Verbindung der Formel (I) in Form enthalten, eine sehr unbefriedigende Lagerstabilität auf. Diese amorphe physikalische Instabilität, die bevorzugt auftritt, wenn die Präparate über einen längeren Zeitraum bei Temperaturen oberhalb 30°C gelagert werden, beeinträchtigt die Resorptionswirksamkeit und vor allem die Sicherheit dieser Präparate. Es ist deshalb von großer Bedeutung eine stabile Form der Verbindung der Formel (I) zur Herstellung von Arzneimitteln zur Verfügung zu stellen, die die oben genannten Nachteile nicht aufweist.

Es wurde nun eine neue kristalline Form der Verbindung (R)-(-)-2-Cycloheptyl-N-methylsulfonyl-[4-(2-chinolinyl-methoxy)-phenyl]-acetamid gefunden, die sich gegenüber der bekannten, nichtkristallinen Form durch erhöhte physikalische Stabilität und verringerte Druckempfindlichkeit auszeichnet und daher für die Herstellung verschiedener Arzneimittelformen wesentlichlich besser geeignet ist als die nichtkristalline Form.

Die Herstellung der erfindungsgemäßen neuen kristallinen Form der Verbindung der Formel (I) erfolgt z.B., in dem man die Verbindung der Formel (I) in nichtkristalliner Form in inerten organischen Lösemitteln, gegebenenfalls in Gegenwart von Wasser suspendiert und bis zur quantitativen Umwandlung in die kristalline Modifikation bei erhöhten Temperaturen behandelt, dann die erhaltenen Kristalle der kristallinen Modifikation nach üblichen Methoden abtrennt und zur Entfernung eventuell vorhandener Lösemittelreste bei Temperaturen von +20°C bis +70°C bis zur Gewichtskonstanz trocknet.

Die Umwandlungsdauer ist abhängig von der Art des Lösemittels und der gewählten Temperatur. Sie beträgt in der Regel mehrere Tage bis Wochen.

Als Lösemittel werden bevorzugt niedrige Alkohole mit 1 bis 6 Kohlenstoffatomen eingesetzt, bevorzugt werden Ethanol oder Isopropanol verwendet. Ganz besonders bevorzugt sind hierbei Mischungen der Alkohole mit Wasser im Verhältnis von einem Teil Alkohol zu 20 Teilen Wasser, bevorzugt von einem Teil Alkohol zu 10 Teilen Wasser. Ganz besonders bevorzugt ist die Verwendung von Ethanol bzw. Isopropanol/Wassergemischen im Verhältnis 1:8.

Die Umwandlung der nichtkristallinen Form in die kristalline Form der Verbindung der Formel (I) erfolgt üblicherweise durch Rühren oder Schütteln der Suspension bei erhöhten Temperaturen.

Das Abtrennen der Kristalle vom Wasser/Lösemittelgemisch geschieht nach den üblichen Methoden, wie beispielsweise durch Filtration, Abdekantieren, Zentrifugieren oder ähnlichem. Anschließend werden die Kristalle bei erhöhter Temperatur, beispielsweise bei +50°C oder im Vakuum bis zur Massenkonstanz getrocknet.

Die vollständige Umwandlung der nichtkristallinen Form in die kristalline Modifikation wird durch DSC (Differential Scanning Calorimetry), spektroskopische oder kristallographische Methoden bestimmt.

Ein schnelleres Verfahren, das sich insbesondere zur Herstellung größerer kristalliner Wirkstoffmengen eignet, gelingt mit Hilfe von Kristallkeimen, indem beispielsweise der nichtkristalline Wirkstoff in einem Gemisch aus Wasser und Ethanol im Verhältnis 8:1 suspendiert wird, die Suspension auf +70°C erwärmt wird, einige Keime des kristallinen Wirkstoffs hinzugegeben werden und ca. 4 h bis zur quantitativen Umwandlung des nichtkristallinen in den kristallinen Wirkstoff gerührt wird. Die vollständige Umwandlung kann jedoch auch unter Verlängerung der Rührzeit bei erhöhter Temperatur unterhalb von +70°C, beispielsweise in einem Temperaturbereich von 40°C bis +60°C, oder in anderen Lösemittelgemischen, wie beispielsweise Wasser und Isopropanol, oder in Lösemittelgemischen mit anderen Mischungsverhältnissen, wie beispielsweise in Gemischen von Wasser und Alkohol im Verhältnis von 15:1 bis 25:1 erfolgen.

Die erfindungsgemäße kristalline Modifikation des (R)-(-)-2-Cycloheptyl-N-methylsulfonyl-[4-(2-chinolinyl-methoxy)-phenyl]-acetamid besitzt ein charakteristisches IR-Spektrum.

Das mittels DSC (Differential Scanning Calorimetry) unter Atmosphärendruck aufgenommene Thermogramm der erfindungsgemäßen kristallinen Modifikation zeigt einen endothermen Schmelzpeak bei 124-127°C und unterscheidet sich damit eindeutig von Thermogrammen anderer Wirkstofformen.

Sowohl die Röntgendiffraktogramme als auch die ¹³C-Festkörper-NMR-Spektren sind für die erfindungsgemäße kristalline Modifikation charakteristisch. Die Dichte des kristallinen (R)-(-)-2-Cycloheptyl-N-methylsulfonyl-[4-(2-chinolinyl-methoxy)-phenyl)-acetamid beträgt 1,30 g/cm³.

Zur vorliegenden Erfindung gehören auch pharmazeutische Zubereitungen, die neben inerten nichttoxischen pharmazeutisch geeigneten Hilfs- und Trägerstoffen die erfindungsgemäße kristalline Form der Verbindung der Formel (I) enthalten oder die aus der kristallinen Modifikation der Verbindung der Formel (I) bestehen, sowie Verfahren zur Herstellung dieser Zubereitungen. Bevorzugt sind hier feste Arzneiformen sowie Suspensionen zu nennen. Besonders geeignet ist der kristalline Wirkstoff zur Herstellung von festen Arzneimitteln, z.B. in Form von Tabletten, Pillen, Dragees oder Kapseln.

Die neue kristalline Modifikation der Verbindung der Formel (I) soll in diesen Zubereitungen in einer Konzentration von 0,1 bis 99,5 Gew.-%, bevorzugt von 0,5 bis 95 Gew.-% der Gesamtmischung vorhanden sein.

Neben der kristallinen Modifikation der Verbindung der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

### Herstellungsbeispiele

### Beispiel 1

### Herstellung von Kristallkeimen

1 g nichtkristalliner Wirkstoff wird in einem Wasser/Isopropanol-Gemisch (8 ml:1 ml) suspendiert und 14 Tage bei + 50°C gerührt. Die Suspension wird anschließend filtriert und bis zur Massenkonstanz im Vakuum getrocknet.

### Beispiel 2

### Kristallisation durch Animpfen

1 g nichtkristalliner Wirkstoff wird in einem Wasser/Ethanol-Gemisch (8 ml:1 ml) suspendiert, mit einigen Kristallkeimen angeimpft und 4 Stunden bei +70°C gerührt. Die Suspension wird anschließend filtriert und bis zur Massenkonstanz getrocknet.

## Patentansprüche

1. (R)-(-)-2-Cycloheptyl-N-methylsulfonyl-[4-(2-chinolinyl-methoxy)-phenyl]acetamid der Formel 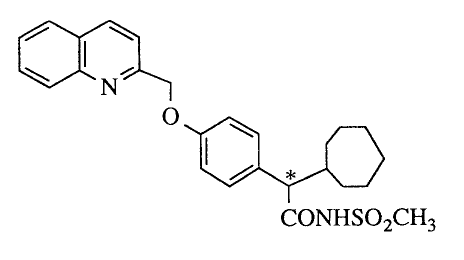 in kristalliner Form.

2. Kristalliner Wirkstoff nach Anspruch 1 zur therapeutischen Anwendung.

3. Verfahren zur Herstellung des kristallinen Wirkstoffes nach Anspruch 1, dadurch gekennzeichnet, daß man nichtkristallines (R)-(-)-2-Cycloheptyl-N-methylsulfonyl-[4-(2-chinolinyl-methoxy)-phenyl]acetamid in inerten organischen Lösemitteln, gegebenenfalls in Gegenwart von Wasser, suspendiert und bis zur quantitativen Umwandlung in die kristalline Modifikation bei erhöhten Temperaturen behandelt, dann die erhaltenen Kristalle der kristallinen Modifikation nach üblichen Methoden abtrennt und zur Entfernung eventuell vorhandener Lösemittelreste bei Temperaturen +20°C bis +70°C bis zur Gewichtskonstanz trocknet.

4. Verfahren nach Anspruch 3, dadurch gekennzeichnet, daß man in Gemischen von niedrigen Alkoholen mit bis zu 6 Kohlenstoffatomen und Wasser arbeitet.

5. Verfahren nach Anspruch 3, dadurch gekennzeichnet, daß man in Ethanol/Wasser- oder Isopropanol/Wassergemischen arbeitet.

6. Verfahren nach Anspruch 3, dadurch gekennzeichnet, daß man mit Alkohol/Wassergemischen im Verhältnis von einem Teil Alkohol zu 20 Teilen Wasser arbeitet.

7. Verwendung des kristallinen Wirkstoffes nach Anspruch 1 zur Herstellung von Arzneimitteln.

8. Verwendung nach Anspruch 7 zur Herstellung von festen Arzneimitteln.

9. Arzneimittel, enthaltend den kristallinen Wirkstoff nach Anspruch 1.

10. Verfahren zur Herstellung von Arzneimittel nach Anspruch 5, dadurch gekennzeichnet, daß man den kristallinen Wirkstoff gegebenenfalls mit Hilfe von üblichen Hilfs- und Trägerstoffen in eine geeignete Applikationsform überführt.
